(19) Europäisches Patentamt
European Patent Office
Office européen des brevets

(11) **EP 2 491 073 B1**

(12) **EUROPÄISCHE PATENTSCHRIFT**

(45) Veröffentlichungstag und Bekanntmachung des Hinweises auf die Patenterteilung:
**19.07.2017 Patentblatt 2017/29**

(21) Anmeldenummer: **10767944.1**

(22) Anmeldetag: **13.10.2010**

(51) Int Cl.:
*C08J 5/18* (2006.01)     *C08J 3/205* (2006.01)
*C08J 3/20* (2006.01)     *C08J 9/00* (2006.01)
*C08K 5/098* (2006.01)     *B01D 67/00* (2006.01)
*B01D 71/16* (2006.01)     *B29C 55/12* (2006.01)
*H01M 2/16* (2006.01)     *C07C 51/41* (2006.01)

(86) Internationale Anmeldenummer:
**PCT/EP2010/006240**

(87) Internationale Veröffentlichungsnummer:
**WO 2011/047797 (28.04.2011 Gazette 2011/17)**

(54) **NANOSKALIGES -NUKLEIERUNGSMITTEL FÜR POLYPROPYLEN**

NANOSCALE -NUCLEATING AGENT FOR POLYPROPYLENE

AGENT NANOMÉTRIQUE DE -NUCLÉATION POUR POLYPROPYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.10.2009 DE 102009050439**

(43) Veröffentlichungstag der Anmeldung:
**29.08.2012 Patentblatt 2012/35**

(73) Patentinhaber: **Treofan Germany GmbH & Co. KG**
**66539 Neunkirchen (DE)**

(72) Erfinder:
• **BUSCH, Detlef**
**66740 Saarlouis (DE)**
• **KLEIN, Dominic**
**66450 Bexbach (DE)**

• **SCHMITZ, Bertram**
**F-57200 Saareguemines (FR)**

(74) Vertreter: **Kremer, Viola**
**Treofan Germany GmbH & Co. KG**
**Am Prime Parc 17**
**65479 Raunheim (DE)**

(56) Entgegenhaltungen:
**WO-A1-02/081557     DE-A1- 4 420 989**

• **ZHANG Z ET AL: "Preparation and characteristics of nano-CaCO3 supported beta-nucleating agent of polypropylene", EUROPEAN POLYMER JOURNAL, PERGAMON PRESS LTD. OXFORD, GB, Bd. 44, Nr. 7, 1. Juli 2008 (2008-07-01), Seiten 1955-1961, XP022853352, ISSN: 0014-3057, DOI: DOI:10.1016/J.EURPOLYMJ.2008.04.022 [gefunden am 2008-04-22]**

**Beschreibung**

Die Erfindung betrifft ein nanoskaliges ß-Nukleierungsmittel für Polypropylen, sowie ein Verfahren zur Erhöhung des Anteils der ß-Kristallmodifikation in Polypropylen, sowie eine poröse Folie.

Von Polypropylen sind neben der amorphen Phase drei verschiedene kristalline, die $\alpha$-, ß-, und $\gamma$-Phasen bekannt. Beim Abkühlen von Polypropylenschmelzen bildet sich üblicherweise überwiegend das $\alpha$-kristalline PP. Durch eine bestimmte Temperaturführung beim Abkühlen einer Polypropylenschmelze kann ein erhöhter Anteil an ß-kristalliner Phase erzeugt werden. Der auf diese Weise erzeugte Anteil an ß-kristallinem PP beträgt weniger als 10 % (1 Aufheizen). Die hexagonale ß-Modifikation des PP's zeichnet sich gegenüber der monoklinen $\alpha$-Modifikation durch bessere mechanische Eigenschaften, z.B. bessere Schlagzähigkeit und Spannungsrißbeständigkeit, aus. Daneben weist die ß-Modifikation des Polypropylens mit 140-155°C einen deutlich niedrigeren Schmelzpunkt gegenüber der $\alpha$-Modifikation mit einem Schmelzpunkt von mindestens 160°C auf. Ein erhöhter Anteil an $\beta$-kristallinem PP wirkt sich daher in einigen Anwendungen günstig auf bestimmte Gebrauchseigenschaften des Polypropylens aus. Aus diesem Grund wurden in der Vergangenheit Additive entwickelt, die beim Abkühlen einer Schmelze zu hohen Anteilen an Polypropylen in der ß-Modifikation führen, sogenannte ß-Nukleierungsmittel oder $\beta$-Nukleatoren.

Als ß-Nukleator mit hoher Aktivität ist der Farbstoff $\gamma$-Quinacridone in dem Deutschen Patent 1188278 beschrieben. Der Nachteil dieses Nukleierungsmittel ist jedoch die intensive Rotfärbung und die mangelnde thermische Stabilität. Im US-Patent 3,540,979 ist das Calciumsalz der Phtalsäure als thermisch stabiles Nukleierungsmittel beschrieben. Der Nachteil dieses Nukleierungsmittel ist die geringe Aktivität. Der damit erzielte Anteil an $\beta$-kristallinen PP beträgt höchsten 70% (K~0,5-0,7).

Ein zweikomponentiges Nukleierungssystem aus Calciumcarbonat und organischen Dicarbonsäuren beschreibt DE 3610644. Dieses Nukleierungssystem zeigt in der Praxis jedoch eine schwankende Aktivität. Der direkte Einsatz der Calciumsalze der in DE 3 610 644 beschriebenen Dicarbonsäuren ist in DE 4 420 989 beschrieben. Die ß-nukleierende Wirkung verschiedener Dicarboxamide, insbesondere N,N-Dicyclohexyl-2,6-Naphtalen-dicarboxamide beschreibt EP-0557721. Nachteil dieses Nukleators sind die hohen Eduktkosten, sowie komplizierte Syntheseschritte bei der Herstellung.

Der Artikel "Preparation and Characteristics of nano-CaCO3 supported ß-nucleating agent of polypropylene" ISSN 0014-3057 beschreibt nanoskalige Partikel aus CaCO3, die mit Pimelinsäure beschichtet werden. Die nano-Partikel werden in Polypropylen eingemischt. Das Polypropylen wird auf aufgeschmolzen, bei dieser Temperatur gehalten und mit einer Kühlrate von 10K/min auf Raumtemperatur abgekühlt. Dabei wird der Träger aus nanoskaligem CaCO3 mit Pimelinsäure beschichtet. Beim Abkühlen der Schmelze, welche diese beschichteten Partikel enthält, entsteht ein erhöhter Anteil an ß-kristallinem Polypropylen.

WO 02/081557 beschreibt ein Verfahren zur Herstellung eines mikroporösen Materials. Es werden Propylenpolymer und ß-Nukleierungsmittel und ein Verdünnungsmittel vermischt. Das Verdünnungmittel ist oberhalb der Schmelzetemperatur mit der Polypropylenschmelze mischbar und bildet unterhalb der Kristallisationstemperatur eine separate Phase. Nach der Abkühlung der Schmelzemischung wird biaxial verstreckt und eine mikroporöse Folie erhalten. Das ß-Nukleierungsmittel ist Calciumpimelat.

Die Aufgabe der vorliegenden Erfindung bestand darin, ein verbessertes ß-Nukleierungsmittel, sowie ein Verfahren zur Erzeugung von ß-kristallinem Polypropylen zur Verfügung zu stellen, sowie ein verbessertes Verfahren zur Herstellung einer Folie mit einer hohen Gasdurchlässigkeit. Mittels dieses Verfahrens sollen hohe ß-Anteile reproduzierbar und zuverlässig erreicht werden können. Das Verfahren soll einfach und effizient durchführbar sein. Die Modifizierung mit einem ß-Nukleierungsmittel darf die üblichen wichtigen Gebrauchseigenschaften des Polypropylens nicht beeinträchtigen. Die Laufsicherheit bei der Herstellung von porösen Folien soll verbessert werden.

Diese Aufgabe wird gelöst durch ein Verfahren zur Herstellung einer stabilen Dispersion aus einer nicht-wässrigen, flüssigen Phase und dispergierten Dicarbonsäuresalzen, bei welchem eine aliphatische Dicarbonsäure mit einem zweiwertigen Metallsalz in einer wässrigen Lösung zu einem Dicarbonsäuresalz umgesetzt wird und das Dicarbonsäuresalz anschließend abgetrennt und getrocknet wird, wobei dieses getrocknete Dicarbonsäuresalz in einer nicht-wässrigen, flüssigen Phase aufgeschlämmt und zerkleinert wird, bis eine stabile Dispersion entsteht, wobei die Dispersion <1% Dicarbonsäuresalze mit einer Teilchengrösse von >1 $\mu$m enthält, wobei die Partikelgrösse und das Vorliegen von Agglomeraten an Rasterelektronischen Mikroskop-Aufnahmen der Proben bestimmt wird. Unter einer Dispersion wird im Sinne der vorliegenden Erfindung ein heterogenes Gemisch verstanden, bei dem das Dicarbonsäuresalz als Feststoff in der kontinuierlichen flüssigen Phase fein verteilt als disperse Phase vorliegt, wobei das Salz nicht oder kaum in der flüssigen Phase gelöst wird. Die beiden Phasen bilden auch keine chemische Verbindung. Die Dispersion zeichnet sich auch dadurch aus, daß die einzelnen Phasen von einander abgegrenzt, d.h. nicht ineinander gelöst, sind und durch physikalische Methoden, z. B. filtern, zentrifugieren, wieder voneinander getrennt werden können. Die stabile Dispersion entmischt sich im wesentlichen nicht mehr, beispielsweise durch sedimentieren, von selbst.

Unter einer nicht-wässrigen Phase wird im Sinne der vorliegenden Erfindung eine organische Verbindung verstanden, die bei Raumtemperatur flüssig ist und deren Wassergehalt <1 Gew.-% beträgt, beispielsweise Alkohole, niedere Alkane, Ketone und ähnliche Flüssigkeiten.

Trocknung bedeutet, je nach gegebenem Zusammenhang, im Sinne der vorliegenden Erfindung sowohl die Entfernung von Wasser oder Feuchte als auch die Abtrennung der nicht-wässrigen, flüssigen Phase.

Diese Aufgabe wird auch gelöst durch ein Compound erhältlich durch ein Verfahren bei welchem die nicht-wässrige flüssige Phase aus der Dispersion hergestellt nach einem der Ansprüche 1 bis 4 entfernt wird, das verbleibende Pulver aus Dicarbonsäuresalz mit Polypropylen vermischt wird und anschließend die so erhaltene Vormischung aufgeschmolzen und zu einem granulierten Compound extrudiert wird.

Unter einem Compound wird im Sinne der vorliegenden Erfindung eine homogene Mischung aus mindestens einem Polypropylen und Dicarbonsäuresalz als Additiv verstanden.

Die Aufgabe wird ebenfalls gelöst durch ein Polypropylen mit einem Anteil an ß-kristallinem Polypropylen, erhältlich nach einem Verfahren, bei welchem die nicht-wässrige, flüssige Phase aus der Dispersion, hergestellt nach einem der Ansprüche 1 bis 4, entfernt wird, das verbleibende Pulver aus Dicarbonsäuresalz mit Polypropylen vermischt wird und bei einer Temperatur von mindestens 150°C aufgeschmolzen und anschließend die Schmelze langsam bei einer Temperatur im Bereich von 60 bis 135°C abgekühlt wird, und die abgekühlte Polypropylenschmelze einen Anteil an ß-kristallinem Polypropylen von 10 bis 95% (1.Aufheizen) aufweist, wobei dieser ß-kristalline Polypropylen-Anteil mittels DSC Messung wie in der Beschreibung unter der entsprechenden Überschrift beschrieben bestimmt wird.

Die Aufgabe wird ebenfalls gelöst durch ein Polypropylen mit einem Anteil an []- kristallinem Polypropylen, erhältlich nach einem Verfahren, bei welchem ein Compound nach Anspruch 5 oder 6, gegebenenfalls gemischt mit Polypropylen ohne Nukleierungsmittel und/oder einem weiteren Polyolefinen und/oder weiteren Zusatzstoffen, bei einer Temperatur von mindestens 150°C aufgeschmolzen und anschließend die Schmelze langsam bei einer Temperatur im Bereich von 60 bis 135°C abgekühlt wird, und die abgekühlte Polypropylenschmelze einen Anteil an ß-kristallinem Polypropylen von 10 bis 95% (1.Aufheizen) aufweist, wobei dieser ß-kristalline Polypropylen-Anteil mittels DSC Messung wie in der Beschreibung unter der entsprechenden Überschrift beschrieben bestimmt wird. Die Aufgabe wird auch gelöst durch eine biaxial verstreckte Polypropylenfolie mit mindestens einer porösen Schicht, erhältlich nach einem Verfahren, bei welchem ein Compound nach Anspruch 5 oder 6, gegebenenfalls gemischt mit weiterem Polypropylen und/oder weiteren Polymeren und/oder weiteren Zusatzstoffen, bei einer Temperatur von mindestens °160°C aufgeschmolzen und durch eine Flachdüse extrudiert und auf Abkühlwalzen bei einer Temperatur im Bereich von 80 bis 130°C, mit einer langen Verweildauer bei dieser Temperatur abkühlt wird , und die abgekühlte Vorfolie mindestens 40% (gemessen nach DSC, 1. Aufheizen) β-kristallines Polypropylen aufweist, wobei dieser ß-kristalline Polypropylen-Anteil mittels DSC Messung wie in der Beschreibung unter der entsprechenden Überschrift beschrieben bestimmt wird, und die Vorfolie anschließend erwärmt und in Längsrichtung und in Querrichtung verstreckt wird und wobei die Temperatur bei der Streckung so gewählt wird, dass sich das ß-kristalline Polypropylen der Vorfolie in die alpha-Modifikation des Polypropylens umwandelt.

Letzlich wird die Aufgabe gelöst durch die Verwendung einer porösen Folie nach einem der Ansprüche 9 bis 12 als Membran in Batterien, sekundären Batterien oder in Superkondensatoren.

Die abhängigen Ansprüche beschreiben bevorzugte Ausführungsformen der Erfindung. Die vorliegende Erfindung beruht auf der Entdeckung, daß nanoskalige Dicarbonsäuresalze beim Abkühlen einer Polypropylen-Schmelze, die diese nanoskaligen Dicarbonsäuresalze enthält, zur Bildung eines hohen Anteils von ß-kristallinem Polypropylen (nachstehend auch ß-Anteil) führen. Die abgekühlte Schmelze mit einem hohen ß-Anteil bildet eine transparente PP-Matrix, da die Partikelgröße der nanoskaligen Dicarbonsäuresalze deutlich kleiner als die Wellenlänge des sichtbaren Lichtes ist. Nanoskalige Dicarbonsäuresalze haben im allgemeinen eine Teilchengröße von 1 bis 500nm, vorzugsweise 5 bis 300nm, wobei gleichzeitig Teilchen oder Agglomerate mit einer Teilchengröße von >1 $\mu$m zu weniger als 3%, vorzugsweise >0 bis <1% enthalten sind. Somit liegt auch die mittlere Teilchengröße der nanoskaligen Dicarbonsäuresalze im besagten Bereich von 1 bis 500nm, vorzugsweise 5 bis 300nm.

Nanoskalige Dicarbonsäuresalze umfassen im Sinne der vorliegenden Erfindung Salze, deren zu Grunde liegende aliphatische Dicarbonsäure mindestens 4 bis 15 C-Atome, insbesondere 5 bis 10 C-Atome aufweist. Besonders bevorzugt sind Salze der Pimelinsäure oder Suberinsäure, beispielsweise Ca-Pimelat oder Ca-Suberat. Es könne auch Gemische verschiedener Dicarbonsäuresalze eingesetzt werden. Im allgemeinen sind Erdalkalisalze bevorzugt, es können jedoch grundsätzlich auch andere zweiwertige Metallsalze, beispielsweise basierend auf zweiwertigem Eisen, Nickel, Zink, etc. eingesetzt werden. Dicarbonsäuresalzes in der flüssigen Phase aufgeschlämmt. Nach dem Aufschlämmen des Salzes in der flüssigen Phase wird der Schlamm vermahlen. Zur Zerkleinerung dienen beispielsweise gängige Mörsermühlen, Ultraschall oder eine Kugelmühle oder andere gängige Nassmahl- oder Zerkleinerungsprozesse. Hierbei wird das Dicarbonsäuresalz vorzugsweise auf eine Partikelgröße von unter 1 bis 500nm, insbesondere 5 bis 200nm zerkleinert. Das nanoskalige Dicarbonsäuresalz bildet nach der Zerkleinerung in der flüssigen Phase eine stabile Dispersion in der Agglomerate von mehr als 1000nm nur in geringen Mengen oder gar nicht mehr vorliegen. Der Übergang zu der nanoskaligen dispergierten Phase zeigt sich auch darin, daß das aufgeschlämmte Dicarbonsäuresalz vor der Mahlung nach Aufschlämmen in der flüssigen Phase zunächst direkt, z.B. innerhalb weniger Minuten, wieder sedimentiert, aber nach der Vermahlung eine stabile, milchige, trübe Dispersion bildet, in der sich die Teilchen nicht mehr absetzen. Diese Dispersion ist somit über eine übliche Zeitdauer bis zur Verarbeitung im wesentlichen stabil, z.B. für eine Dauer von mindestens einer oder auch mehreren Stunden. Gegebenenfalls kann die Schlämme zusätzlich filtriert werden, um solche Agglomerate abzutrennen, die nach der Mahlung noch vorhanden sind. Das Filtermedium wird so gewählt, daß alle Teilchen mit einer Größe von >1$\mu$m abgetrennt werden und die Schlämme anschließend frei von Teilchen dieser Größe ist oder zumindest davon weniger als 1% enthält.

Diese stabile Dispersion kann entweder direkt mit dem Polypropylen, z.B. in Form von Pulver oder Granulat, vermischt und getrocknet werden. Alternativ wird die flüssige Phase der Dispersion abgetrennt und das so erhaltene Pulver aus nanoskaligem Dicarbonsäuresalz mit Polypropylen, in Form von Pulver oder Granulat, vermischt. Über diese beiden möglichen Verfahrensvarianten erhält man eine Vormischung aus nanoskaligen Dicarbonsäuresalzen und Polypropylen. Die Abtrennung der nicht-wässrigen flüssigen Phase erfolgt in beiden Verfahren mit üblichen geeigneten Mitteln, beispielsweise durch Verdunsten, Abziehen im Vakuum, Abdestillieren oder mittels einer Filterpresse. Die Vormischungen enthalten im allgemeinen 0 bis 2 Gew.-% der flüssigen Phase, vorzugsweise >0 bis 1 Gew.-%.

Gegebenenfalls kann zusätzlich zur noch besseren Vermeidung von Agglomeration der nanoskaligen Dicarbonsäuresalze und zur Verbesserung der Dispergierbarkeit der Dicarbonsäuresalze in der Polypropylen-Matrix, entweder bei der Herstellung der Dispersion oder beim Mischen der Dicarbonsäuresalze mit Polypropylen eine oberflächenaktive Substanz, wie z.B. höherwertige Carbonsäuren, Silane, Amine oder Sulfonate zugesetzt werden. Besonders bevorzugt sind für diese Zwecke langkettige Fettsäuren, wie Ölsäure oder Stearinsäure. Überraschenderweise ist die erfindungsgemäße Dispersionen jedoch auch ohne solche Hilfsmittel weitgehend stabil.

Anschließend können diese Vormischungen aus Polypropylen und Dicarbonsäuresalz direkt zu Produkten verarbeitet werden, wobei gegebenenfalls weitere Polyolefine und/oder weitere Zusatzstoffe zugesetzt werden können. In einer bevorzugten Variante werden diese Vormischungen in einem weiteren Verfahrensschritt zu Granulatkörnern mit nanoskaligen Dicarbonsäuresalzen compoundiert. Die Herstellung des Compounds erfolgt in üblicher Weise in dem die Vormischung bei geeigneten Temperaturen aufgeschmolzen wird, beispielsweise in einem Bereich von 160 bis 300°C. Das Aufschmelzen erfolgt vorzugsweise in einem geeigneten Extruder, beispielsweise in einem Zweischneckenextruder, welcher gleichzeitig eine gute Mischung des nanoskaligen Dicarbonsäuresalzes im Polypropylen gewährleistet. Die aufgeschmolzene Mischung wird zu Granulatkörnern extrudiert und diese bei geeigneten Temperaturen abgekühlt. Bei der Compoundierung können ebenfalls zusätzlich zu dem Polypropylen weitere Zusatzstoffe und/oder andere Polyolefine hinzugefügt werden, beispielsweise Polyethylene. Diese Compounds werden dann bei der Herstellung der Produkte, beispielsweise Spritzgußteile, Folien, poröse Folien, Fasern etc. eingesetzt.

Im Allgemeinen enthalten die Vormischungen, bzw. die daraus granulierten Compounds 0,0001 bis 5 Gew.-%, vorzugsweise 0,001 bis 3 Gew.-% nanosklalige, aliphatische Dicarbonsäuresalze. Insbesondere für Folienanwendungen ist ein Gehalt von 0,001 bis 1 Gew.-% Dicarbonsäuresalze in dem Compound, bzw. der Vormischung bevorzugt. Die Angaben in Gew.-% beziehen sich jeweils auf das Gewicht der Mischung, bzw. des Compounds. Gegebenfalls können auch verschiedene Dicarbonsäuresalze gemischt und anschließend verwendet werden.

Die Vormischungen oder die Compounds aus mindestens einem Polypropylen und nanoskaligen Dicarbonsäuresalzen, die bei der Herstellung der Produkte eingesetzt werden, enthalten im allgemeinen mindestens 50 bis <100 Gew.-%, vorzugsweise 60 bis 99 Gew.-%, insbesondere 70 bis 99 Gew.-%, eines Polypropylens und gegebenenfalls weitere Polyolefine, wie z.B. Polyethylene und/oder weitere Zusatzstoffe. Die Angaben in Gew.-% beziehen sich jeweils auf das Gewicht der Mischung.

Geeignete Polypropylene sind beispielsweise isotaktische Propylenhomopolymere mit einem Schmelzpunkt von 140 bis 170°C, vorzugsweise von 155 bis 168°C, und einen Schmelzflußindex (Messung DIN 53 735 bei 21,6 N Belastung und 230°C) von 1,0 bis 50g/10min, vorzugsweise von 1,5 bis 20g/10min. Der n-heptanlösliche Anteil des Polymeren beträgt im allgemeinen 1 bis 10 Gew.-%, vorzugsweise 2 bis 5 Gew.-% bezogen auf das Ausgangspolymere. Die Molekulargewichtsverteilung des Propylenpolymeren kann variieren.

Das Verhältnis des Gewichtsmittels $M_w$ zum Zahlenmittel $M_n$ liegt im allgemeinen zwischen 1 und 15, vorzugsweise bei 2 bis 10, ganz besonders bevorzugt bei 2 bis 6. Eine derartig enge Molekulargewichtsverteilung des Propylenhomopolymeren erreicht man beispielsweise durch dessen peroxidischen Abbau oder durch Herstellung des Polypropylens mittels geeigneter Metallocenkatalysatoren.

In einer weiteren Ausführungsform der Erfindung ist das eingesetzte Propylenhomopolymer hochisotaktisch. Bei derartigen hochisotaktischen Polypropylenen beträgt der mittels $^{13}$C-NMR-Spektroskopie bestimmte Kettenisotaxie-Index des n-heptanunlöslichen Anteils des Polypropylens mindestens 95 %, vorzugsweise 96 bis 99 %.

Des weiteren sind als Polypropylene Propylenmischpolymerisate geeignete, welche im allgemeinen mindestens 80 Gew.-%, vorzugsweise 90 bis <100 Gew.-%, insbesondere 95 bis 99 Gew.-%, Propyleneinheiten enthalten. Der entsprechende Comonomergehalt von höchstens 20 Gew.-% bzw. >0 bis 10 Gew.-% bzw. 1 bis 5 Gew.-% besteht, wenn vorhanden, im allgemeinen aus Ethylen und/oder Butylen. Die Angaben in Gew.-% beziehen sich jeweils auf das Propylenpolymere. Geeignete Mischpolymerisate, welche z.B. Ethylen und/oder Butylen als Comonomer enthalten, sind vorzugsweise statistische Mischpolymerisate oder Blockcopolymere.

4

Nach dem erfindungsgemäßen Verfahren zur Herstellung von Polypropylen mit einem erhöhten Anteil an ß-kristallinem Polypropylen wird die Vormischung aus Polypropylen und nanoskaligen Dicarbonsäuresalzen oder das Compound bei geeigneten Temperaturen aufgeschmolzen. Diese Temperatur liegt im allgemeinen in einem Bereich von 160 bis 300°C. Das Aufschmelzen erfolgt vorzugsweise in einem geeigneten Extruder, beispielsweise in einem Zweischneckenextruder, welcher gleichzeitig eine gute Mischung des nanoskaligen Dicarbonsäuresalzes im Polypropylen gewährleistet. Die aufgeschmolzene Mischung wird extrudiert und bei geeigneten Temperaturen abgekühlt.

Sowohl die Vormischung als auch die Compounds können in dem erfindungsgemäßen Verfahren zusammen mit weiteren Polypropylenen ohne Nukleierungsmittel und /oder gegebenenfalls mit weiteren Polyolefinen und/oder Zusatzstoffen eingesetzt werden. Alle Komponenten werden dann gemeinsam in einem beliebigen Extrusionswerkzeug oder in einem Kneter aufgeschmolzen und miteinander vermischt und zu Produkten mit einem Anteil an ß-kristallinem Polypropylen extrudiert.

Es ist bei allen Verfahrensvarianten erfindungswesentlich, daß nach der Extrusion die Abkühlung der Schmelze, welche nanosklaige Dicarbonsäuresalze enthält, derart erfolgt, daß die ß-nukleierende Wirkung der nanoskaligen Dicarbonsäuresalze zum Tragen kommt. Hierfür ist es bevorzugt die Schmelze langsam bei einer Temperatur in einem Bereich von 60 bis 135°C, vorzugsweise bei 80 bis 130°C abzukühlen. Je näher diese Temperatur in der Nähe der Kristallisationstemperatur des ß-kristallinen Polypropylens liegt, um so günstiger sind die Bedingungen für die Ausbildung der ß-kristallinen Modifikation. Auf diese Weise kann über die Auswahl der Temperatur beim Abkühlen ein mehr oder weniger hoher Anteil an ß-Polypropylen erzeugt werden. Zusätzlich hat die Verweildauer der abkühlenden Schmelze bei der jeweiligen Temperatur einen Einfluß auf den erzielten ß-Anteil. Zur Erzielung eines größtmöglichen ß-Anteils sollte die Schmelze sehr langsam bei den höheren Temperaturen abgekühlt werden, wobei die notwendige Verweildauer bei der gegebenen Temperatur im Einzelfall von der Formgebung bei der Extrusion abhängt.

Je nach Anwendungsfall können auch niedrigere ß-Anteile im Polypropylen ausreichend sein. Die ß-nukleierenden Dicarbonsäuresalze wirken sich in diesen Fällen positiv aus, da die Abkühlrate erhöht werden kann, d.h. schnellere Abzugs- oder Extrusionsgeschwindigkeiten eingesetzt werden können. Der ß-Anteil (1. Aufheizen) des nach diesem Verfahren hergestellten Polypropylens kann somit je nach Anwendung im Bereich von 10 - 95%, vorzugsweise 20 - 80%, insbesondere 50 - 90% variieren.

Mittels des erfindungsgemäßen Verfahrens ist es möglich bei entsprechenden Abkühlbedingungen einen Gehalt an ß-Polypropylen von >80%, vorzugsweise 85 bis 95% (DSC-Methode, 1. Aufheizen) zu erzielen. Beispielsweise wurden über DSC Messungen (1. Aufheizen) an isotaktischem Propylenhomopolymer mit 0,1 Gew.-% nanoskaligem Dicarbonsäuresalze ein Anteil von ß-kristallinem Polypropylen von 92 % bestimmt.

Das erfindungsgemäße Verfahren kann vorteilhaft bei der Herstellung von Folien, Formkörpern, insbesondere Rohren und Schläuchen, Fasern und anderen Extrusionsprodukten angewendet werden. Die hohe Effizienz der nanoskaligen ß-Dicarbonsäuren wirkt sich bei den verschiedensten Extrusionsanwendungen günstig aus, beispielsweise da die Extrusionstemperatur reduziert oder die Verweildauer verkürzt werden kann. Für einige Anwendungen ist ein erhöhter Anteil an ß-kristallinem Polypropylen vorteilhaft, da hierdurch Gebrauchseigenschaften des Polypropylens verbessert werden, z.B. erreicht man eine höhere Kerbschlagzähigkeit und Spannungsrißbeständigkeit des Polypropylens. In einer weiteren Anwendung nutzt man den besonders hohe ß-Anteile im Polypropylen zur Herstellung von porösen Folien durch Umwandlung der ß-Modifikation in die alpha-Modifikation bei der Verstreckung von Folien oder zur Erzeugung von rauhen Oberflächen einer verstreckten Folie aus.

Es wurde gefunden, daß das nanoskalige Dicarbonsäuresalz überraschende Vorteile bei der Verwendung in einem Verfahren zur Herstellung einer porösen biaxialen verstreckten Folie oder auch einer verstreckten Folie mit einer oder mehreren porösen Schichten bietet. Zum einen wirken sich die hohen Gehalte an ß-Polypropylen positiv auf die Porosität dieser Folie, bzw. der porösen Schicht, und deren Gasdurchlässigkeit aus. Es wurde jedoch auch gefunden, daß andere ß-Nukleierungsmittel zu vergleichbar hohen ß-Gehalten in der Vorfolie führen können, beispielsweise auch Dicarbonsäuresalze, welche bei der Herstellung nicht der zusätzlichen Vermahlung der Schlämme unterworfen werden. Aber es zeigt sich, daß bei Verwendung derselben das Polypropylen nicht in der gleichen Weise zu Folien, bzw. zu Schichten mit hohen Porositäten verstreckt werden kann. Bei Verwendung der erfindungsgemäßen nanoskaligen Dicarbonsäuren können Streckbedingungen, insbesondere hohe Streckfaktoren angewendete werden, die zu einer besonders hohen Porosität der Folie, bzw. der Schicht führen, wobei gleichzeitig eine überraschend gute Laufsicherheit der Folie gegeben ist.

Die Erfindung ist bei der Herstellung von einschichtigen und mehrschichtigen porösen Folien von Vorteil. Eine Membran-Folie zeichnet sich dadurch aus, daß sie nur eine oder im Falle mehrerer Schichten nur poröse Schichten umfaßt und eine hohe Gasdurchlässigkeit aufweist. Gegebenenfalls kann die Erfindung auch bei einer mehrschichtigen Folie genutzt werden, die neben einer oder mehreren porösen Schicht/en auch eine weitere oder mehrere im wesentlichen gas-undurchlässige Schicht/en umfaßt. Die Angaben in dieser Beschreibung bezüglich der porösen Folie gelten somit sinngemäß in gleicher Weise oder analog auch für die poröse Schicht oder die porösen Schichten einer mehrschichtigen Folie.

Im Einzelnen werden bei der Herstellung einer biaxial verstreckten Polypropylenfolie die Komponenten der porösen Schicht oder Schichten, d.h. die Mischung oder das Compound aus dem nano-Dicarbonsäuresalz und Polypropylen, gegebenenfalls gemischt mit weiterem Polypropylen und/oder weiteren Polymeren und/oder weiteren Zusatzstoffen, in einem Extruder bei einer Temperatur von mindestens 160°C aufgeschmolzen. Die ein- oder mehrschichtige Polymerschmelze wird durch eine Flachdüse co/extrudiert, von einer Abnahmewalze aufgenommen und auf der Abnahmewalze derart abgekühlt, daß sich die Schmelze zu einer Vorfolie verfestigt und der gewünschte Anteil an ß-kristallinem Polypropylen entsteht. Diese Abkühlung der Schmelze erfolgt wie vorstehend bereits beschrieben in einem Temperaturbereich von vorzugsweise 80 bis 130°C, wobei eine lange Verweildauer bei dieser Temperatur zu einem erhöhten ß-Polypropylen-Anteil beiträgt. Für die Herstellung einer porösen Folien, bzw. Schicht wird im allgemeinen ein Anteil von mindestens 40%, vorzugsweise 60 bis 95% an ß-Polypropylen in der Vorfolie (gemessen nach DSC, 1.Aufheizen) angestrebt, wohingegen zur Erzeugung von Oberflächenrauhigkeiten geringere Anteile von beispielsweise 10 bis 40% ausreichend sein können. Anschließend wird die Vorfolie in an sich bekannter Weise erwärmt und in Längsrichtung verstreckt, vorzugsweise bei einer Temperatur weniger als 140°C, insbesondere 80 bis 125°C und mit einem Streckfaktor von 2,5:1 bis 6:1. Nach der Längsstreckung wird die längsgestreckte Folie erneut erwärmt und in Querrichtung verstreckt, vorzugsweise bei einer Temperatur größer 110°C, insbesondere von 120 bis 145°C und mit einem Streckverhältnis von 3:1 bis 8:1. Durch die gewählten Temperaturen bei der Verstreckung wandelt sich das ß-kristalline Polypropylen der Vorfolie in die alpha Modifikation des Polypropylens um und erzeugt je nach Verfahrensbedingungen und ß-Anteil in der Vorfolie eine durchgehende poröse netzwerkartige Struktur in der Folie, bzw. in der porösen Schicht oder zumindest eine Oberflächenrauhigkeit durch kraterartige Vertiefungen, die bei den Umwandlungsprozeßen entstehen. Derartige rauhe Oberflächenstrukturen sind beispielsweise bei Folien mit papierähnlichem Charakter oder bei Kondensatorfolien erwünscht, welche als Dielektrikum in Kondensatoren eingesetzt werden. Um die elektrischen Eigenschaften solcher Kondensatorfolien nicht zu beeinträchtigen ist es bevorzugt das nanoskalige Dicarbonsäuresalz nur in der oder den Deckschicht/en einzusetzen, welche die Oberflächenrauheit aufweisen sollen. Es wurde gefunden, daß die nano-skaligen Dicarbonsäuresalze die elektrischen Eigenschaften der Kondensatorfolie nicht oder nur geringfügig beeinträchtigen.

Überraschenderweise weißt die Folie, bzw. die Schicht, welche mit den erfindungsgemäßen nanoskaligen Dicarbonsäuresalzen hergestellt ist eine, sehr hohe und gleichmäßige Porosität und eine gute mechanische Festigkeit auf. Die gleichmäßige Verteilung der Porengröße ist in REM-Aufnahmen gut zu erkennen. Der mittlere Porendurchmesser (Bubble Point) liegt im Bereich von 50 bis 350nm, bevorzugt im Bereich 60 bis 300nm. Bei der Herstellung der porösen Folie, bzw. der Folie mit poröser Schicht, kommt es nur sehr selten zu Abrissen, d.h. das Verfahren hat eine hohe Laufsicherheit. Die Folie kann mit sehr hohen Faktoren verstreckt werden, so daß außergewöhnlich hohe Porositäten erzielt werden können. Grundsätzlich kann der Gurley-Wert der verschiedenen Ausführungsformen der Folie in einem breiten Bereich variieren. Für solche Folien, welche nur poröse Schichten umfassen und beispielsweise als Membran-Folien verwendet werden, liegt der Gurley Wert im allgemeinen in einem Bereich von 100 - 5000s; vorzugsweise 100 bis 2000s. Überraschenderweise lassen sich nach der vorliegenden Erfindung durch hohe Streckfaktoren auch poröse Folien mit sehr niedrigen Gurley Werten von 10 bis <100s, vorzugsweise 15 bis 80s, insbesondere 15 bis 50s noch verfahrenssicher herstellen. Derartig niedrige Gurley Werte von unter 50 s können mit allen bekannten Verfahren nach dem Stand der Technik nicht erreicht werden. Auch lassen sich poröse Folien mit Gurley Werten <600s und Porositäten >50% mit einer Dicke unter 30 $\mu$m, vorzugsweise 10 - 25$\mu$m, insbesondere 12 - 20$\mu$m noch laufsicher herstellen.

Die porösen Folie, bzw. die poröse/n Schicht/en der Folie enthält/enthalten in einer weiteren Ausführungsform zusätzlich zu den nanoskaligen Dicarbonsäuresalzen und dem vorstehend beschriebenen Polypropylenen als zusätzliche Komponente ein Propylenblockcopolymer, sowie gegebenenfalls weitere Polyolefine, welche die Porosität nicht beeinträchtigen. In diesen Ausführungsformen enthält die Folie bzw. die poröse Schicht im allgemeinen 50 bis 85 Gew.-%, vorzugsweise 60 bis 75 Gew.-%, Propylenhomopolymere und 15 bis 50 Gew.-% Propylenblockcopolymere, vorzugsweise 25 bis 40 Gew.-%, und 0,001 bis 5 Gew.-%, vorzugsweise 50 - 10.000 ppm des nanoskaligen Dicarbonsäuresalzes als ß-Nukleierungsmittels, bezogen auf das Gewicht der porösen Schicht, bzw. bezogen auf das Gewicht der Folie. Gegebenenfalls sind zusätzlich übliche Additive in geringen Mengen von unter 2 Gew.-%, beispielsweise Stabilisatoren und Neutralisationsmittel enthalten. Für den Fall, daß weitere Polyolefine enthalten sind, wird der Anteil des Propylenhomopolymeren oder des Blockcopolymeren entsprechend reduziert. Im allgemeinen wird die Menge der zusätzlichen Polymeren 0 bis <50 Gew.-%, vorzugsweise 0,5 bis 40 Gew.-%, insbesondere 1 bis 30 Gew.-% betragen, wenn diese zusätzlich enthalten sind. In diesen Fällen wird der vorstehend beschriebene Anteil an Polypropylenen oder Propylenblockcopolymeren entsprechend erniedrigt. In gleicher Weise gilt, daß der besagte Propylenpolymer- oder Propylenblockcopolymer-Anteil reduziert wird, wenn höhere Mengen von bis zu 2 Gew.-% Nukleierungsmittel eingesetzt werden.

Die poröse Folie kann einschichtig oder mehrschichtig sein. Die Dicke der porösen Folie liegt im allgemeinen in einem Bereich von 10 bis 200$\mu$m, vorzugsweise 15 bis 150$\mu$m, insbesondere 15 bis 100$\mu$m. Die Dichte der porösen Folie liegt im allgemeinen in einem Bereich von 0,1 bis 0,6 g/cm$^3$, vorzugsweise 0,2 bis 0,5 g/cm$^3$. Die poröse Folie kann mit einer Corona, Flamm- oder Plasmabehandlung versehen werden, um die Befüllung mit Elektrolyten zu verbessern. Gegebenenfalls kann die mikroporöse Folie eine Abschaltschicht umfassen, welche die Durchlässigkeit der Folie bei erhöhten Temperaturen herabsetzt.

Die porösen Folien können vorteilhaft als Membran verwendet werden, beispielsweise in Batterien, sekundären Batterien, in Superkonderisatoren oder in ähnlichen Anwendungen.

Zur Charakterisierung der Rohstoffe und der Folien wurden die folgenden Meßmethoden benutzt:

Schmelzflußindex

Der Schmelzflußindex der Propylenpolymeren wurde nach DIN 53 735 bei 2,16 kg Belastung und 230 °C gemessen und bei 190°C und 2,16 kg für Polyethylene.

Schmelzpunkte

Bei der DSC-Messung wird dem Polymeren mit einer definierten Aufheizrate eine Wärmemenge pro Zeiteinheit zugeführt und der Wärmestrom gegen die Temperatur aufgetragen. Der Schmelzpunkt ist im Sinne der vorliegenden Erfindung das Maximum der DSC Kurve. Zur Bestimmung des Schmelzpunkts wird die DSC-Kurve mit einer Aufheiz- und Abkühlgeschwindigkeit von 10K/1min im Bereich von 20 bis 200°C aufgenommen. Für die Bestimmung des Schmelzpunkts der Polymeren wird wie üblich die zweite Aufheizkurve ausgewertet.

Dichte

Die Dichte $\rho$ wird nach DIN 53 479, Verfahren A, bestimmt.

Porosität

Die Porosität errechnet sich aus der der an der porösen Folie bestimmten Dichte $\rho_F$ und der Dichte des Ausgangsrohstoffes Polyproylen wie folgt:

$$P\ [\%] = 100 \times (1 - \rho_F) / \rho_{PP}$$

Dabei wurde für Polypropylen eine Dichte von $0,92 g/cm^3$ angenommen.

Permeabilität (Gurley-Wert)

Die Permeabilität der Folien wurde mit dem Gurley Tester 4110, nach ASTM D 726-58 gemessen. Dabei wird die Zeit (in sec) bestimmt die $100\ cm^3$ Luft benötigen, um durch die Etikettenfläche von $1\ Inch^2$ ($6,452\ cm^2$) zu permeieren. Die Druckdifferenz über der Folie entspricht dabei dem Druck einer Wassersäule von 12,4 cm Höhe. Die benötigte Zeit entspricht dann dem Gurley-Wert.

ß-Gehalt

Der Anteil des ß-kristallinen Polypropylens wird mittels DSC bestimmt. Diese Charakterisierung wird in J. o. Appl. Polymer Science, Vol. 74, p.: 2357-2368, 1999 von Varga beschrieben und folgendermaßen durchgeführt: Die mit dem ß-Nukleator additivierte Probe wird in der DSC zunächst mit einer Aufheizrate von 20°C/min auf 220°C erhitzt und aufgeschmolzen (1. Aufheizen). Danach wird sie mit einer Kühlrate von 10°C/min auf 100°C abgekühlt, bevor sie mit einer Heizrate von 10°C/min (2. Aufheizen) wieder aufgeschmolzen wird.

Aus der DSC Kurve des 1. Aufheizen wird aus dem Verhältnis der Schmelzenthalpien der ß-kristallinen Phase ($H_ß$) zu der Summe der Schmelzenthalpien von ß- und $\alpha$-kristalliner Phase ($H_ß + H_\alpha$) der Kristallinitätsgrad $K_{ß,DSC}$ ( Anteil an ß-kristallinem Polypropylen) bestimmt, der in der vermessenen Probe vorliegt (unverstreckte Folie, Spritzgussteil). Der prozentuale Wert errechnetsich wie folgt:

$$K_{ß,DSC}\ [\%] = 100 \times (H_ß)/(H_ß + H_\alpha)$$

Aus der DSC Kurve des 2. Aufheizen wird aus dem Verhältnis der Schmelzenthalpien der ß-kristallinen Phase ($H_ß$) zu der Summe der Schmelzenthalpien von ß- und $\alpha$-kristalliner Phase ($H_ß + H_\alpha$) der Kristallinitätsgrad $K_{ß,DSC}$ (2. Aufheizen) bestimmt, der den ß-Anteil der jeweiligen Polypropylenprobe angibt, der maximal erreicht werden kann.

Agglomerate und Teilchengröße

Die Teilchengröße der Dicarbonsäuresalze und das Vorliegen von Agglomeraten wird an Rasterelektronischen Mikroskop-Aufnahme (REM) der Proben bestimmt.

Zur Durchführung der REM-Aufnahmen an einer Folienprobe wird ein Zuschnitt von 5x5mm aus der biaxial verstreckten Folie ausgeschnitten und auf den Probenträger aufgeklebt. Anschließend wird in einer Sputtereinheit eine wenige Nanometer dicke Schicht eines Edelmetalls (Pt, Au, Pd..) auf die Oberfläche der Folie aufgebracht.

Die besputterte Probe wird dann über eine Schleuse in das REM eingebracht und dort im Hochvakuum mit einer Beschleunigungsspannung von mehreren kV abgescannt. Die Beschleunigungsspannung wird so gewählt, dass ein scharfes Bild entsteht, ohne daß sich die Folien-Matrix auf Grund thermischer Belastung deformiert. Die Teilchen sind in der Aufnahme so gut zu erkennen, daß die Größe der einzelnen Partikel mit Hilfe des Maßstabs ausgemessen werden kann.

Die entsprechende Bestimmung der Teilchengröße der Dicarbonsäuresalze im Compound erfolgt an einer Cast-Folie als Probekörper. Hierfür wird aus dem Compound eine ca. 120 bis 150$\mu$m unverstreckte Castfolie hergestellt. Mit dieser Cast-Folie wird verfahren wie oben beschrieben.

Die Folie, bzw. das Compound ist im Sinne der vorliegenden Erfindung frei von Agglomeraten, wenn in der REM-Aufnahme der Folien-Probe keine Teilchen mit einer Größe von mehr als einem 1$\mu$m gefunden werden oder wenn maximal ein Teilchen von > 1$\mu$m vorhanden ist. Die mittlere Teilchengröße kann durch Ausmessen der Teilchengröße einer statisch ausreichenden Anzahl von Teilchen erfolgen. Entsprechend kann auch der Anteil an Agglomeraten von >1$\mu$m an Hand der REM Aufnahmen bestimmt werden.

Zur Bestimmung der Teilchengröße der Dicarbonsäuresalze in Dispersion wird eine geringe Menge der Dispersion auf einem Objektträger aufgetragen, getrocknet und gleichfalls besputtert. An dieser besputterten Probe kann ein REM aufgenommen und die Teilchengröße ermittelt werden. An dieser so vorbereiteten Proben wird auch das Vorliegen von Agglomeraten geprüft.

Die Erfindung wird nun an Hand von Ausführungsbeispielen näher erläutert:

Beispiel 1:

Es wurde eine wäßrige Lösung mit 40g Pimelinsäure in 1000ml Wasser vorgelegt und auf 83°C erwärmt bis die Pimelinsäure vollständig gelöst war. Zu dieser Lösung wurde eine wäßrige Calciumhydroxid-Milch (18,4g Ca(OH)$_2$ in 200ml Wasser) unter Rühren hinzugefügt, wodurch Calciumpimelat als weißer Niederschlag ausfiel. Der sedimentierte Niederschlag wurde abgenutscht und bei 130°C im Trockenschrank vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Calcium-Pimelat erhalten.

100 g dieses getrockneten Calcium-Pimelats wurden in 500ml wasserfreiem (Wassergehalt <1Gew.-%) Isopropanol aufgeschlämmt und die Schlämme in eine Kugelmühle gegeben und vermahlen. Dabei entstand eine stabile milchartige Dispersion. REM Aufnahmen zeigen eine Teilchengröße der Teilchen in der Dispersion im Bereich von 75nm. Es wurden in den Proben keine Agglomerate mit einer Teilchengröße von mehr als 0,8$\mu$m gefunden

Beispiel 1 a:

Die milchartige Dispersion nach Beispiel 1 wurde unter Feuchtigkeitsausschluss bei 90°C für 10h im Ablufttrockner getrocknet. Es wurde ein weißes Pulver aus nanoskaligem Calcium-Pimelat erhalten. Dieses Pulver wurde im Mischer in einer Konzentration von 0,4 Gew.-% bezogen auf das Polypropylen, mit Granulat aus isotaktischem Polypropylenhomopolymer (Schmelzpunkt 162°C; MFI 3g/10min) vermischt. Diese Mischung wurde in einem Zweischneckenextruder aufgeschmolzen (Gehäusetemperatur von 240°C und 200 1/min$^{-1}$) und zu Stäbchen-förmigen Körnern granuliert.

REM Aufnahmen an den Granulatkörnern (Probekörper Cast-Folie) zeigen das feinverteilte agglomeratfreie Calciumpimelat in der PP-Matrix. Es wurden in den REM Aufnahmen keine Teilchen mit einer Größe von >1$\mu$m gefunden. Mittels DSC-Analyse zeigt das Compound aus Polypropylen und nanoskaligem Calciumpimelat einen ß-Wert von 97% beim 2.Aufheizen.

Beispiel 1 b

Die milchartige Dispersion nach Beispiel 1 wurde direkt auf eine Granulat aus isotaktischem Polypropylenhomopolymer aufgetrommelt und diese Mischung beim Auftrommeln (oder anschließend) getrocknet. Nach der Trocknung sind die Granulatkörner mit einer Schicht aus nanoskaligem Calcium-Pimelat überzogen und zeigen eine milchige weiße Farbe.

REM Aufnahmen an diesen Granulatkörnern (Probekörper Cast Folie) zeigen feinverteiltes agglomeratfreies Calcium-Pimelat auf der Oberfläche der Granulatkörner. Diese beschichteten Granulatkörner wurden in einem Zweischneckenextruder aufgeschmolzen (Gehäusetemperatur von 240°C und 200 1/min$^{-1}$) und zu Stäbchen-förmigen Körnern granuliert. Mittels DSC-Analyse zeigt dieses Compound aus Polypropylen und nanoskaligem Calciumpimelat ebenfalls einen ß-Wert von 97% beim 2.Aufheizen. REM Aufnahmen an diesen Granulatkörnern zeigen die Ca-Pimelatpartikel feinverteilt mit einer Größe von < 100 nm. Es wurden in den REM Aufnahmen keine Teilchen mit einer Größe von > 1$\mu$m gefunden.

Vergleichsbeispiel 1

Es wurde eine wäßrige Lösung mit 40g Pimelinsäure in 1000ml Wasser vorgelegt und auf 83°C erwärmt bis die Pimelinsäure vollständig gelöst war. Zu dieser Lösung wurde eine wäßrige Calciumhydroxid-Lösung (18,4g Ca(OH)$_2$ in 200ml) unter Rühren hinzugefügt, wodurch Calciumpimelat als weißer Niederschlag ausfiel. Der sedimentierte Niederschlag wurde abgenutscht und bei 130°C im Trockenscharnk vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Calcium-Pimelat erhalten.
100 g dieses getrockneten Calcium-Pimelats wurden in eine Kugelmühle gegeben und in trockenem Zustand vermahlen. Es wurde ein weißes Pulver aus Calcium-Pimelat erhalten. REM Aufnahmen zeigen eine Korngröße des Pulvers im Bereich von 500nm mit Agglomeraten deren Teilchengröße bis zu 2$\mu$m beträgt.

Vergleichsbeispiel 1a:

Das Pulver gemäß Vergleichsbeispiel 1 wurde im Mischer in einer Konzentration von 0,4 Gew.-% mit Granulat aus isotaktischem Polypropylenhomopolymer (Schmelzpunkt 162°C; MFI 3g/10min) vermischt. Diese Mischung wurde in einem Zweischneckenextruder aufgeschmolzen (Gehäusetemperatur von 240°C und 200 1/min$^{-1}$) und zu Stäbchen-förmigen Körnern granuliert.
REM Aufnahmen an den Granulatkörnern (Probekörper Cast Folie) zeigen das feinverteilte Calcium-Pimelat in der PP-Matrix, jedoch sind auch Agglomerate mit einer Teilchengröße von 1 bis 10$\mu$m vorhanden. Mittels DSC-Analyse zeigt die Mischung aus Polypropylen und nanoskaligem Calciumpimelat einen ß-Wert von 97% beim 2.Aufheizen.

Folienbeispiel 1

In einem Mischer wurden das Compound nach Beispiel 1 a mit Propylenhomopolymer und Propylenblockcopolymer vermischt. Diese Mischung wurde in einem Extruder aufgeschmolzen und weiter homogenisiert. Nach dem Extrusionsverfahren wurde die Schmelze aus einer Breitschlitzdüse bei einer Extrusionstemperatur von 245 °C zu einer einschichtigen Folie extrudiert. Diese Folie hatte die folgende Zusammensetzung:

| ca. 50 Gew.-% | Propylenhomopolymerisat (PP) mit einem n-heptanlöslichen Anteil von 4,5 Gew.-% (bezogen auf 100 % PP) und einem Schmelzpunkt von 165 °C; und einem Schmelzflußindex von 3,2 g/10 min bei 230 °C und 2,16 kg Belastung (DIN 53 735) und |
| ca. 49,96 Gew.-% | Propylen-Ethylen-Blockcopolymerisat mit einem Ethylenanteil von ca. 5 Gew.-% bezogen auf das Blockcopolymer und einem Schmelzflußindex (230°C und 2,16 kg) von 6 g/10min |
| 0,04 Gew.-% | nano Ca- Pimelat als ß-Nukleierungsmittel |

Die Folie enthielt zusätzlich Stabilisator und Neutralisationsmittel in üblichen Mengen.
Die Polymermischung wurde nach der Extrusion über eine erste Abzugswalze und ein weiteres Walzentrio abgezogen, abgekühlt und verfestigt, anschließend längsgestreckt, quergestreckt und fixiert, wobei im einzelnen die folgenden Bedingungen gewählt wurden:

| Extrusion: | Extrusionstemperatur 245 °C |
| Abkühlwalze: | Temperatur 125°C, |
| Abzugsgeschwindigkeit: | 1,5 m/min (Verweilzeit auf der Abzugswalze: 55 sec) |
| Längsstreckung: | Streckwalze T = 90 °C |
| Längsstreckung um den | Faktor 4 |

(fortgesetzt)

| Querstreckung: | Aufheizfelder T = 145 °C |
| Streckfelder | T = 145 °C |
| Querstreckung um den | Faktor 4 |

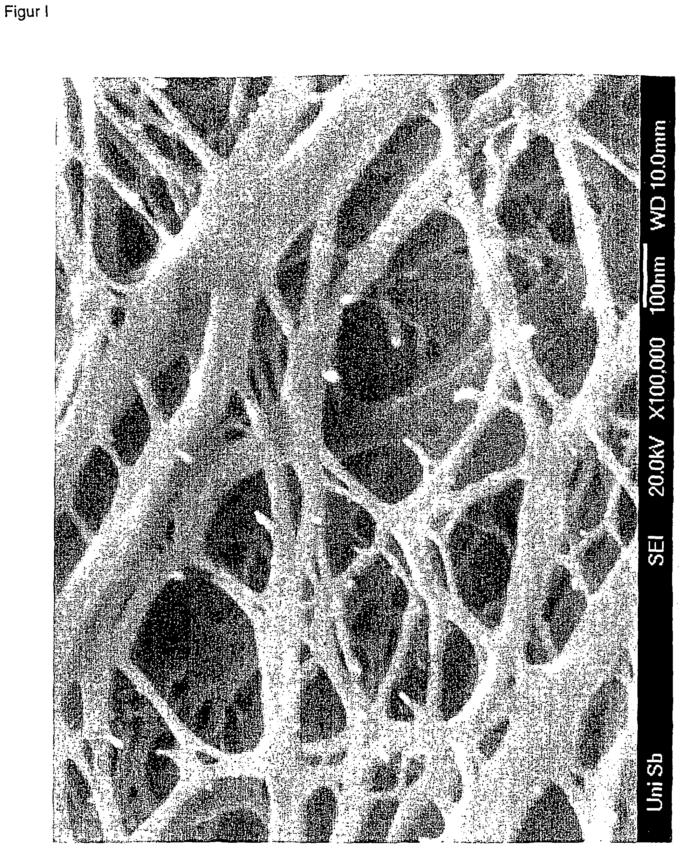

Die so hergestellte poröse Folie war ca. 30 $\mu$m dick und wies eine Dichte von 0,30 g/cm$^3$ auf und zeigte ein gleichmäßiges weiß-opakes Aussehen. Die Porosität betrug 66% und der Gurley-Wert 340 s. Bei der Folienherstellung traten über mehrere Stunden keine Abrisse auf. Die REM Aufnahme (Figur 1) zeigt eine gleichmäßige Verteilung des Ca-Pimelats ohne Agglomerate in der porösen Folie. Die Dicarbonsäuresalze sind gut erkennbar als helle Flecken an den Polymersträngen des Netzwerks aus Polypropylen.

Folienbeispiel 2

Es wurde eine Folie wie in Folienbeispiel 1 beschrieben hergestellt. Im Unterschied zu Folienbeispiel 1 wurden jetzt das Compound nach Beispiel 1 b eingesetzt. Es wurde eine Folie mit den gleichen Eigenschaften wie nach Beispiel 1 erhalten. Es traten gleichfalls keine Abrisse bei der Herstellung auf.

Folienbeispiel 3

Es wurde eine Folie wie in Folienbeispiel 2 beschrieben hergestellt. Die Zusammensetzung wurde nicht geändert. Im Unterschied zu Folienbeispiel 1 wurde bei der Herstellung mit einem Längsstreckfaktor von 4,8 und bei der Querstreckung mit einem Faktor von 5,8 verstreckt. Die so hergestellte poröse Folie war ca. 20 $\mu$m dick und wies eine Dichte von 0,25 g/cm$^3$ auf und zeigte ein gleichmäßiges weiß-opakes Aussehen. Die Porosität betrug 60 % und der Gurley-Wert 200 s. Es traten gleichfalls keine Abrisse bei der Herstellung auf.

Folienbeispiel 4

Es wurde eine Folie wie in Folienbeispiel 3 beschrieben hergestellt. Die Zusammensetzung wurde nicht geändert. Im Unterschied zu Folienbeispiel 1 wurde bei der Herstellung eine niedrigere Abzugsgeschwindigkeit von 1 m/min (Verweilzeit auf der Abzugswalze: 80 sec) gewählt. Die übrigen Verfahrensbedingungen wurden nicht geändert. Die so hergestellte poröse Folie war ca. 25 $\mu$m dick und wies eine Dichte von 0,25 g/cm$^3$ auf und zeigte ein gleichmäßiges weiß-opakes Aussehen. Die Porosität betrug 70 % und der Gurley-Wert 60s. Auch diese Folie ließ sich überraschend verfahrenssicher herstellen.

Vergleichsbeispiel 1 (Folie)

Es wurde eine Folie wie in Folienbeispiel 1 beschrieben hergestellt. Es wurde jedoch im Unterschied zu Folienbeispiel 1 ein Compound hergestellt nach Vergleichsbeispiel 1a eingesetzt. Es wurde eine Folie mit einem ähnlichen Eigenschaftsprofil erhalten. Es kam jedoch im Laufe von 4 Produktionsstunden zu 5 Abrissen. REM Aufnahmen an der biaxial verstreckten Folie zeigen agglomerierte Teilchen in einer Größe von bis zu 5$\mu$m.

Vergleichsbeispiel 2:

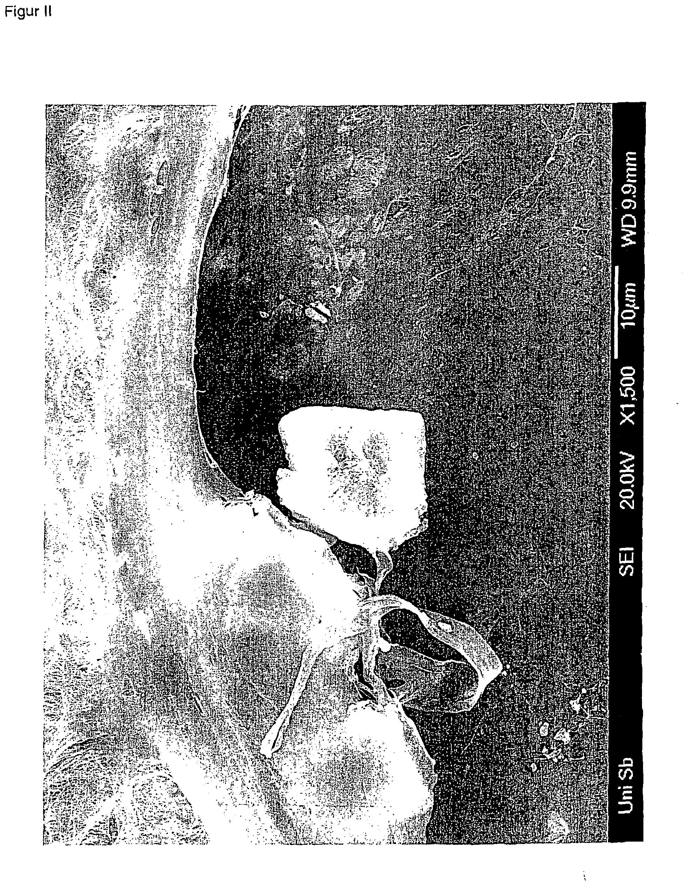

Es wurde eine Folie wie in Folienbeispiel 3 hergestellt. Es wurde jedoch im Unterschied zu Folienbeispiel 3 ein Compound hergestellt nach Vergleichsbeispiel 1a eingesetzt. Es wurde eine Folie mit einem ähnlichen Eigenschaftsprofil wie nach Folienbeispiel 3 erhalten. Es kam jedoch im Laufe von 4 Produktionsstunden zu 10 Abrissen. De facto war die Folie nicht verfahrenssicher herstellbar und unwirtschaftlich. REM Aufnahmen (Figur 2) zeigt agglomerierte Teilchen in einer Größe von bis zu 5$\mu$m und größer. In Figur 2 ist insbesondere gut zu erkennen wie diese Agglomarate Aufriße beim Verstrecken erzeugen, die zu Abrißen bei der Herstellung führen.

Vergleichsbeispiel 3:

Es wurde versucht eine Folie wie in Folienbeispiel 4 hergestellt. Es wurde jedoch im Unterschied zu Folienbeispiel 4 ein Compound hergestellt nach Vergleichsbeispiel 1 a eingesetzt. Es konnte mit diesen Verfahrensbedingungen auf Grund ständiger Abrisse keine Folie hergestellt werden.

Beispiel 2:

Es wurde eine wäßrige Lösung mit 40g Suberinsäure in 1000ml Wasser vorgelegt und auf 85°C erwärmt bis die Suberinsäure vollständig gelöst war. Zu dieser Lösung wurde eine wäßrige Calciumhydroxid-Milch (17,02g Ca(OH)$_2$ in 200ml Wasser) unter Rühren hinzugefügt, wodurch Calciumsuberat als weißer Niederschlag ausfiel. Der sedimentierte Niederschlag wurde abgenutscht und bei 130°C im Trockenschrank vorgetrocknet. Abschließend wurde bei 200°C für 24h in einem Vakuumtrockenschrank die Restfeuchte und Kristallwasser entfernt. Auf diese Weise wurde ein grobkörniges getrocknetes Pulver aus Calcium-Pimelat erhalten.

100 g dieses getrockneten Calcium-Suberats wurden in 300ml wasserfreiem Isopropanol aufgeschlämmt und die Schlämme in eine Kugelmühle gegeben und vermahlen. Dabei entstand eine stabile milchartige Dispersion. REM Aufnahmen zeigen eine Teilchengröße der Teilchen in der Dispersion im Bereich von 75nm. Es wurden in den Proben keine Agglomerate mit einer Teilchengröße von mehr als 1μm gefunden

Beispiel 2a: Pulver

Die milchartige Dispersion nach Beispiel 2 wurde unter Feuchtigkeitsausschluss bei 90°C für 10h im Ablufttrockner getrocknet. Es wurde ein weißes Pulver aus nanoskaligem Calcium-Suberat erhalten.

Dieses Pulver wurde im Mischer in einer Konzentration von 0,4 Gew.-% bezogen auf das Polypropylen, mit Granulat aus isotaktischem Polypropylenhomopolymer (Schmelzpunkt 162°C; MFI 3g/10min) vermischt. Diese Mischung wurde in einem Zweischneckenextruder aufgeschmolzen (Gehäusetemperatur von 240°C und 200 1/min$^{-1}$) und zu Stäbchen-förmigen Körnern granuliert.

REM Aufnahmen an den Granulatkörnern (Probekörper Cast Folie) zeigen das feinverteilte agglomeratfreie Calciumsuberat in der PP-Matrix. Mittels DSC-Analyse zeigt die Mischung aus Polypropylen und nanoskaligem Calciumsuberat einen ß-Wert von 99% beim 2. Aufheizen.

Beispiel 2b

Die milchartige Dispersion nach Beispiel 2 wurde direkt auf eine Granulat aus isotaktischem Polypropylenhomopolymer aufgetrommelt und diese Mischung beim Auftrommeln (oder anschließend) getrocknet. Nach der Trocknung sind die Granulatkörner mit einer Schicht aus nanoskaligem Calcium-Suberat überzogen und zeigen eine milchige weiße Farbe.

REM Aufnahmen an den Granulatkörnern (Probekörper Cast Folie) zeigen feinverteiltes agglomeratfreies Calcium-Suberat auf der Oberfläche der Granulatkörner. Diese beschichteten Granulatkörner wurden in einem Zweischneckenextruder aufgeschmolzen (Gehäusetemperatur von 240°C und 200 1/min$^{-1}$) und zu Stäbchen-förmigen Körnern granuliert. Mittels DSC-Analyse zeigt diese Compound aus Polypropylen und nanoskaligem Calciumsuberat ebenfalls einen ß-Wert von 99% beim 2.Aufheizen. REM Aufnahmen an den Granulatkörnern (Probekörper Cast-Folie) zeigen die Ca-Suberatpartikel feinverteilt mit einer Größe von <100 nm. Es liegen keine Agglomerate mit einer Teilchengröße von >1μm vor.

Folienbeispiel 5

In einem Mischer wurden das Compound nach Beispiel 2a mit Propylenhomopolymer und Propylenblockcopolymer vermischt. Diese Mischung wurde in einem Extruder aufgeschmolzen und weiter homogenisiert. Nach dem Extrusionsverfahren wurde die Schmelze aus einer Breitschlitzdüse bei einer Extrusionstemperatur von 245 °C zu einer einschichtigen Folie extrudiert. Diese Folie hatte die folgende Zusammensetzung:

| | |
|---|---|
| ca. 50 Gew.-% | Propylenhomopolymerisat (PP) mit einem n-heptanlöslichen Anteil von 4,5 Gew.-% (bezogen auf 100 % PP) und einem Schmelzpunkt von 165 °C; und einem Schmelzflußindex von 3,2 g/10 min bei 230 °C und 2,16 kg Belastung (DIN 53 735) und |
| ca. 49,96 Gew.-% | Propylen-Ethylen-Blockcopolymerisat mit einem Ethylenanteil von ca. 5 Gew.-% bezogen auf das Blockcopolymer und einem Schmelzflußindex (230°C und 2,16 kg) von 6 g/10min |
| 0,04 Gew.-% | nano Ca- Suberat als ß-Nukleierungsmittel |

Die Folie enthielt zusätzlich Stabilisator und Neutralisationsmittel in üblichen Mengen.

Die Polymermischung wurde nach der Extrusion über eine erste Abzugswalze und ein weiteres Walzentrio abgezogen, abgekühlt und verfestigt, anschließend längsgestreckt, quergestreckt und fixiert, wobei im einzelnen die folgenden Bedingungen gewählt wurden:

| | |
|---|---|
| Extrusion: | Extrusionstemperatur 245 °C |
| Abkühlwalze: | Temperatur 125°C, |
| Abzugsgeschwindigkeit: | 1,5 m/min (Verweilzeit auf der Abzugswalze: 55 sec) |
| Längsstreckung: | Streckwalze T = 90 °C |
| Längsstreckung um den | Faktor 4 |
| Querstreckung: | Aufheizfelder T = 145 °C |
| Streckfelder | T = 145 °C |
| Querstreckung um den | Faktor 4 |

Die so hergestellte poröse Folie war ca. 30 $\mu$m dick und wies eine Dichte von 0,30 g/cm$^3$ auf und zeigte ein gleichmäßiges weiß-opakes Aussehen. Die Porosität betrug 66% und der Gurley-Wert 340 s. Bei der Folienherstellung traten über mehrere Stunden keine Abrisse auf.

Folienbeispiel 6

Es wurde eine Folie wie in Folienbeispiel 5 beschrieben hergestellt. Im Unterschied zu Folienbeispiel 5 wurden jetzt das Compound nach Beispiel 2b eingesetzt. Es wurde eine Folie mit den gleichen Eigenschaften wie nach Folienbeispiel 5 erhalten. Es traten gleichfalls keine Abrisse bei der Herstellung auf.

**Patentansprüche**

1.   Verfahren zur Herstellung einer stabilen Dispersion aus einer nicht-wässrigen flüssigen Phase und dispergierten Dicarbonsäuresalzen, bei welchem eine aliphatische Dicarbonsäure mit einem zweiwertigen Metallsalz in einer wässrigen Lösung zu einem Dicarbonsäuresalz umgesetzt wird und das Dicarbonsäuresalz anschließend abgetrennt und getrocknet wird, **dadurch gekennzeichnet, dass** anschließend das getrocknete Dicarbonsäuresalz in einer nicht-wässrigen, flüssigen Phase aufgeschlämmt und zerkleinert wird, bis eine stabile Dispersion entsteht, wobei die Dispersion <1% Dicarbonsäuresalze mit einer Teilchengrösse von >1 $\mu$m enthält, wobei die Teilchengrösse und das Vorliegen von Agglomeraten an Rasterelektronsichen Mikroskop-Aufnahmen der Proben bestimmt wird.

2.   Verfahren nach Anspruch 1, **dadurch gekennzeichnet, dass** die Dicarbonsäure eine aliphatische Dicarbonsäure mit 4 bis 15 C-Atome ist und die nicht-wässrige, flüssige Phase ein Alkohol ist.

3.   Verfahren nach Anspruch 1 oder 2, **dadurch gekennzeichnet, dass** die Dicarbonsäure Pimelinsäure oder Suberinsäure ist und das Metallsalz ein Erdalkalisalz ist.

4.   Verfahren nach einem der Ansprüche 1 bis 3, **dadurch gekennzeichnet, dass** das Metallsalz ein Hydroxid, ein Carbonat oder ein Chlorid ist.

5.   Compound aus Polypropylen, erhältlich durch ein Verfahren, bei welchem die nicht-wässrige, flüssige Phase aus der Dispersion, hergestellt nach einem der Ansprüche 1 bis 4, entfernt wird, das verbleibende Pulver aus Dicarbonsäuresalz mit Polypropylen vermischt wird und anschließend die so erhaltene Vormischung aufgeschmolzen und zu einem granulierten Compound extrudiert wird.

6.   Compound aus Polypropylen, erhältlich durch ein Verfahren, bei welchem eine Dispersion, hergestellt nach einem der Ansprüche 1 bis 4, mit Polypropylen vermischt wird, die nicht-wässrige, flüssige Phase aus dieser Mischung entfernt wird und anschließend die so erhaltene Vormischung aufgeschmolzen und zu einem granulierten Compound extrudiert wird.

7.   Polypropylen mit einem Anteil an $\beta$- kristallinem Polypropylen, erhältlich nach einem Verfahren, bei welchem die nicht-wässrige, flüssige Phase aus der Dispersion, hergestellt nach einem der Ansprüche 1 bis 4, entfernt wird, das verbleibende Pulver aus Dicarbonsäuresalz mit Polypropylen vermischt wird und bei einer Temperatur von mindestens 150°C aufgeschmolzen und anschließend die Schmelze langsam bei einer Temperatur im Bereich von 60 bis 135°C abgekühlt wird, und die abgekühlte Polypropylenschmelze einen Anteil an β-kristallinem Polypropylen von 10 bis 95% aufweist, wobei dieser β-kristalline Polypropylen-Anteil mittels DSC Messung wie in der Beschreibung unter der entsprechenden Überschrift beschrieben bestimmt wird.

8. Polypropylen mit einem Anteil an β- kristallinem Polypropylen, erhältlich nach einem Verfahren, bei welchem ein Compound nach Anspruch 5 oder 6, gegebenenfalls gemischt mit Polypropylen ohne Nukleierungsmittel und/oder einem weiteren Polyolefinen und/oder weiteren Zusatzstoffen, bei einer Temperatur von mindestens 150°C aufgeschmolzen und anschließend die Schmelze langsam bei einer Temperatur im Bereich von 60 bis 135°C abgekühlt wird, und die abgekühlte Polypropylenschmelze einen Anteil an β-kristallinem Polypropylen von 10 bis 95% aufweist, wobei dieser β-kristalline Polypropylen-Anteil mittels DSC Messung wie in der Beschreibung unter der entsprechenden Überschrift beschrieben bestimmt wird.

9. Biaxial verstreckte Polypropylenfolie mit mindestens einer porösen Schicht, erhältlich nach einem Verfahren, bei welchem ein Compound nach Anspruch 5 oder 6, gegebenenfalls gemischt mit weiterem Polypropylen und/oder weiteren Polymeren und/oder weiteren Zusatzstoffen, bei einer Temperatur von mindestens 160°C aufgeschmolzen und durch eine Flachdüse extrudiert und auf Abkühlwalzen bei einer Temperatur im Bereich von 80 bis 130°C mit einer langen Verweildauer bei dieser Temperatur, abkühlt wird , und die abgekühlte Vorfolie mindestens 40% β-kristallines Polypropylen aufweist, wobei dieser β-kristallin Polypropylen-Anteil mittels DSC Messung wie in der Beschreibung unter der entsprechenden Überschrift beschrieben, bestimmt wird, und die Vorfolie anschließend erwärmt und in Längsrichtung und in Querrichtung verstreckt wird und wobei die Temperatur bei der Streckung so gewählt wird, dass sich das β-kristalline Polypropylen der Vorfolie in die α-Modifikation des Polypropylens umwandelt.

10. Polypropylenfolie nach Anspruch 9, **dadurch gekennzeichnet, dass** das nanoskalige Dicarbonsäuresalz in der porösen Folie eine Teilchengröße von weniger als 100 nm aufweist und keine Agglomerate mit einer Teilchengröße von > 1 μm vorliegen.

11. Polypropylenfolie nach Anspruch 9 oder 10, **dadurch gekennzeichnet, dass** die Folie einen Gurley Wert von <500 s aufweist, gemessen nach ASTM D 726-58.

12. Polypropylenfolie nach einem der Ansprüche 9 bis 11, **dadurch gekennzeichnet, dass** die Porosität der Folie > 50% ist, wobei die Porosität P mittels Dichtemessung nach DIN 53 479, Verfahren A, wie in der Beschreibung unter der entsprechenden Überschrift beschrieben, bestimmt wird.

13. Verwendung einer porösen Folie nach einem der Ansprüche 9 bis 12 als Membran in Batterien, sekundären Batterien oder in Superkondensatoren.

14. Biaxial verstreckte Polypropylenfolie mit mindestens einer Schicht, erhältlich nach einem Verfahren, bei welchem für die Schicht ein Compound, hergestellt nach einem der Anspruch 5 bis 6, gegebenenfalls gemischt mit weiterem Polypropylen und/oder weiteren Polymeren und/oder weiteren Zusatzstoffen, bei einer Temperatur von mindestens 160°C aufgeschmolzen und durch eine Flachdüse extrudiert und auf Abkühlwalzen bei einer Temperatur im Bereich von 80 bis 130°C, mit einer langen Verweildauer bei dieser Temperatur, abkühlt wird und die abgekühlte Vorfolie in der Schicht einen 10 bis 40% β-kristallinenes Polypropylen aufweist, wobei dieser β-kristalline Polypropylen-Anteil mittels DSC Messung wie in der Beschreibung unter der entsprechenden Überschrift beschrieben bestimmt wird, und die Vorfolie anschließend erwärmt und in Längsrichtung und in Querrichtung verstreckt wird und und wobei die Temperatur bei der Streckung so gewählt wird, dass sich das β-kristalline Polypropylen der Vorfolie in die α-Modifikation des Polypropylens umwandelt und die Schicht eine äußere Schicht der Folie bildet und diese Schicht auf der äußeren Oberfläche eine Oberflächenrauheit durch kraterartige Vertiefungen aufweist.

15. Verwendung einer Folie nach Anspruch 14 als Dielektrikum in Kondensatoren oder als Folie mit papier-ähnlichen Eigenschaften.

## Claims

1. A process for the production of a stable dispersion formed from a non-aqueous liquid phase and dispersed dicarboxylic acid salts, in which an aliphatic dicarboxylic acid is reacted with a divalent metallic salt in an aqueous solution in order to form a dicarboxylic acid salt and the dicarboxylic acid salt is subsequently separated and dried, **characterized in that** subsequently, the dried dicarboxylic acid salt is elutriated and comminuted in a non-aqueous liquid phase until a stable dispersion is formed, wherein the dispersion contains < 1 % of dicarboxylic acid salts with a particle size of > 1 $\mu$m, wherein the particle size and the presence of agglomerates are determined by means of scanning electron microscope images of samples.

2. The process according to claim 1, **characterized in that** the dicarboxylic acid is an aliphatic dicarboxylic acid containing 4 to 15 C atoms and the non-aqueous liquid phase is an alcohol.

3. The process according to claim 1 or 2, **characterized in that** the dicarboxylic acid is pimelic acid or suberic acid and the metallic salt is an alkaline-earth salt.

4. The process according to one of claims 1 to 3, **characterized in that** the metallic salt is a hydroxide, a carbonate or a chloride.

5. A blend formed from polypropylene, which can be obtained by means of a process in which the non-aqueous liquid phase is removed from the dispersion produced according to one of claims 1 to 4, the remaining dicarboxylic acid salt powder is admixed with polypropylene and subsequently, the pre-mixture obtained thereby is melted and extruded to form a granular blend.

6. A blend formed from polypropylene, which can be obtained by means of a process in which a dispersion produced according to one of claims 1 to 4 is admixed with polypropylene, the non-aqueous liquid phase is removed from said admixture, and subsequently the pre-mixture obtained in this manner is melted and extruded to form a granular blend.

7. Polypropylene containing a fraction of $\beta$-crystalline polypropylene, which can be obtained by means of a process in which the non-aqueous liquid phase is removed from the dispersion produced according to one of claims 1 to 4, the remaining dicarboxylic acid salt powder is admixed with polypropylene and melted at a temperature of at least 150 °C and subsequently, the melt is slowly cooled at a temperature in the range 60 to 135 °C, and the cooled polypropylene melt contains a fraction of $\beta$-crystalline polypropylene of 10 to 95 %, wherein said $\beta$-crystalline polypropylene fraction is determined using DSC measurements as described in the description under the relevant heading.

8. Polypropylene containing a fraction of $\beta$-crystalline polypropylene, which can be obtained by means of a process in which a blend according to claim 5 or 6, optionally admixed with polypropylene without a nucleation agent and/or an additional polyolefin and/or additional additives, is melted at a temperature of at least 150 °C and subsequently, the melt is slowly cooled at a temperature in the range 60 to 135 °C, and the cooled polypropylene melt contains a fraction of $\beta$-crystalline polypropylene of 10 to 95 %, wherein said $\beta$-crystalline polypropylene fraction is determined using DSC measurements as described in the description under the relevant heading.

9. A biaxially stretched polypropylene film having at least one porous layer, which can be obtained by means of a process in which a blend claim 5 or 6, optionally mixed with additional polypropylene and/or additional polymers and/or additional additives, is melted at a temperature of at least 160 °C and extruded through a flat nozzle and cooled on cooling rollers at a temperature in the range 80 to 130 °C with a long dwell time at said temperature, and the cooled pre-film contains at least 40 % $\beta$-crystalline polypropylene, wherein said $\beta$-crystalline polypropylene fraction is determined using DSC measurements as described in the description under the relevant heading, and the pre-film is subsequently heated and stretched in the longitudinal direction and in the transverse direction, and wherein the temperature during stretching is selected such that the $\beta$-crystalline polypropylene of the pre-film is transformed into the $\alpha$-modification of the polypropylene.

10. The polypropylene film according to claim 9, **characterized in that** the nanoscale dicarboxylic acid salt in the porous film has a particle size of less than 100 nm and no agglomerates with a particle size of > 1 $\mu$m are present.

11. The polypropylene film according to claim 9 or 10, **characterized in that** the film has a Gurley value of < 500 s, measured in accordance with ASTM D 726-58.

12. The polypropylene film according to one of claims 9 to 11, **characterized in that** the porosity of the film is > 50 %, wherein the porosity P is determined by measurement of the density in accordance with DIN 53 479, Method A, as described in the description under the relevant heading.

**13.** Use of a porous film according to one of claims 9 to 12, as a membrane in batteries, secondary batteries or in supercapacitors.

**14.** A biaxially stretched polypropylene film with at least one layer which can be obtained in accordance with a process in which, as the layer, a blend produced according to claim 5 to 6, optionally mixed with additional polypropylene and/or additional polymers and/or additional additives, is melted at a temperature of at least 160 °C and extruded through a flat nozzle and cooled on cooling rollers at a temperature in the range 80 to 130 °C, with a long dwell time at that temperature, and the cooled pre-film in the layer contains 10 to 40 % β-crystalline polypropylene, wherein said β-crystalline polypropylene fraction is determined using DSC measurements as described in the description under the relevant heading, and the pre-film is then heated and stretched in the longitudinal direction and in the transverse direction, and wherein the temperature during stretching is selected in a manner such that the β-crystalline polypropylene of the pre-film is transformed into the α-modification of the polypropylene and the layer forms an outer layer of the film and the outer surface of said layer has a surface roughness formed by crater-like depressions.

**15.** Use of a film according to claim 14, as a dielectric in capacitors or as a film with paper-like properties.

**Revendications**

**1.** Procédé de préparation d'une dispersion stable à partir d'une phase liquide non-aqueuse et de sels d'acide dicarboxylique en dispersion, consistant à faire réagir un acide dicarboxylique dans une solution aqueuse avec un sel métallique divalent pour obtenir un sel d'acide dicarboxylique, ledit sel d'acide dicarboxylique étant ensuite séparé et séché, **caractérisé en ce que**, suite à son séchage, le sel d'acide dicarboxylique est mis en suspension dans une phase liquide non-aqueuse et broyé jusqu'à l'obtention d'une dispersion stable, ladite dispersion contenant < 1 % de sels d'acide dicarboxylique ayant une granulométrie > 1 μm, la granulométrie et la présence d'agglomérés étant déterminées à l'aide de clichés d'échantillons obtenus par microscopie électronique à balayage.

**2.** Procédé selon la revendication 1, **caractérisé en ce que** ledit acide dicarboxylique est un acide dicarboxylique aliphatique renfermant 4 à 15 atomes de carbone, et ladite phase liquide non-aqueuse est un alcool.

**3.** Procédé selon les revendications 1 ou 2, **caractérisé en ce que** l'acide dicarboxylique est de l'acide pimélique ou de l'acide subérique, et ledit sel métallique est un sel alcalino-terreux.

**4.** Procédé selon l'une des revendications 1 à 3, **caractérisé en ce que** le sel métallique est un hydroxyde, un carbonate ou un chlorure.

**5.** Formulation complète à base de polypropylène, pouvant être obtenue par un procédé qui consiste à enlever la phase liquide non-aqueuse de la dispersion préparée selon l'une des revendications 1 à 4, à mélanger le résidu pulvérulent de sel d'acide dicarboxylique avec du polypropylène puis à faire fondre le pré-mélange ainsi obtenu et l'extruder de manière à récupérer une formulation complète sous forme de granulés.

**6.** Formulation complète à base de polypropylène, pouvant être obtenue par un procédé qui consiste à mélanger la dispersion préparée selon l'une des revendications 1 à 4 avec du polypropylène, à enlever la phase liquide non-aqueuse de ce mélange, puis à faire fondre le pré-mélange ainsi obtenu et l'extruder de manière à récupérer une formulation complète sous forme de granulés.

**7.** Polypropylène comportant une proportion de polypropylène β-cristallin, lequel peut être obtenu selon un procédé consistant à enlever la phase liquide non-aqueuse de la dispersion préparée selon l'une des revendications 1 à 4, à mélanger le résidu pulvérulent de sel d'acide dicarboxylique avec du polypropylène et à le faire fondre à une température d'au moins 150 °C puis à refroidir la matière en fusion lentement à une température comprise entre 60 et 135 °C, cette matière de polypropylène fondue comportant suite à son refroidissement une proportion de polypropylène β-cristallin comprise entre 10 et 95 %, ladite proportion de polypropylène β-cristallin étant déterminée au moyen d'une mesure par calorimétrie différentielle à balayage telle que décrite sous le titre correspondant.

**8.** Polypropylène comportant une proportion de polypropylène β-cristallin, lequel peut être obtenu selon un procédé consistant à faire fondre une formulation complète selon les revendications 5 ou 6, le cas échéant en mélange avec du polypropylène exempt d'agents de nucléation et/ou avec d'autres polyoléfines et/ou avec d'autres additifs, à une température d'au moins 150 °C puis à refroidir la matière en fusion lentement à une température comprise entre 60 et 135 °C, cette matière de polypropylène fondue comportant suite à son refroidissement une proportion de polypropylène β-cristallin comprise entre 10 et 95 %, ladite proportion de polypropylène β-cristallin étant déterminée au moyen d'une mesure par calorimétrie différentielle à balayage telle que décrite sous le titre correspondant.

9. Feuille de polypropylène biaxialement orientée laquelle comporte au moins une couche poreuse et laquelle peut être obtenue selon un procédé consistant à faire fondre une formulation complète selon les revendications 5 ou 6, le cas échéant en mélange avec du polypropylène supplémentaire et/ou d'autres polymères et/ou d'autres additifs, à une température d'au moins 160 °C et à l'extruder à travers une filière plate et à la refroidir sur des cylindres de refroidissement à une température comprise entre 80 et 130 °C, le temps de séjour à cette température étant long, la pré-feuille comportant suite à son refroidissement au moins 40 % de polypropylène β-cristallin, ladite proportion de polypropylène β-cristallin étant déterminée au moyen d'une mesure par calorimétrie différentielle à balayage telle que décrite sous le titre correspondant, pour ensuite réchauffer ladite pré-feuille et l'orienter dans la direction longitudinale et la direction transversale, la température durant cette orientation étant choisie de manière à ce que le polypropylène β-cristallin de la pré-feuille se transforme en la modification α du polypropylène.

10. Feuille de polypropylène selon la revendication 9, **caractérisée en ce que** le sel d'acide dicarboxylique à l'échelle nano présente, au sein de ladite feuille poreuse, une granulométrie inférieure à 100 nm tout en étant exempt d'aggloméré d'une granulométrie > 1 $\mu$m.

11. Feuille de polypropylène selon les revendications 9 ou 10, **caractérisée en ce que** la feuille présente un indice de Gurley < 500 s, mesuré selon ASTM D 726-58.

12. Feuille de polypropylène selon l'une des revendications 9 à 11, **caractérisée en ce que** la porosité de ladite feuille est > 50 %, la porosité P étant déterminée par mesure de la densité selon DIN 53 479, méthode A, telle qu'exposée dans la description sous le titre correspondant.

13. Utilisation d'une feuille poreuse selon l'une des revendications 9 à 12 en tant que membrane dans des batteries, des accumulateurs électriques ou dans des supercondensateurs.

14. Feuille de polypropylène biaxialement orientée laquelle comportant au moins une couche et laquelle peut être obtenue selon un procédé consistant réaliser cette couche en faisant fondre une formulation complète préparée selon l'une des revendications 5 à 6, le cas échéant en mélange avec du polypropylène supplémentaire et/ou d'autres polymères et/ou d'autres additifs, à une température d'au moins 160 °C, et à l'extruder à travers une filière plate et à la refroidir sur des cylindres de refroidissement à une température comprise entre 80 et 130 °C, le temps de séjour à cette température étant long, la pré-feuille comportant suite à son refroidissement dans ladite couche 10 à 40 % de polypropylène β-cristallin, ladite proportion de polypropylène β-cristallin étant déterminée au moyen d'une mesure par calorimétrie différentielle à balayage telle que décrite sous le titre correspondant, pour ensuite réchauffer ladite pré-feuille et l'orienter dans la direction longitudinale et la direction transversale, la température durant cette orientation étant choisie de manière à ce que le polypropylène β-cristallin de la pré-feuille se transforme en la modification a du polypropylène, et ladite couche formant une couche extérieure de ladite feuille, et ladite couche conférant à la surface extérieure une rugosité de surface occasionnée par des cavités ressemblant à des cratères.

15. Utilisation d'une feuille selon la revendication 14 en tant que diélectrique dans des condensateurs ou en tant que feuille ayant des propriétés proches de celles du papier.

Figur I

Figur II

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1188278
- US 3540979 A
- DE 3610644
- DE 4420989
- EP 0557721 A
- WO 02081557 A

**In der Beschreibung aufgeführte Nicht-Patentliteratur**

- *Preparation and Characteristics of nano-CaCO3 supported ß-nucleating agent of polypropylene,* ISSN 0014-3057
- *J. o. Appl. Polymer Science,* 1999, vol. 74, 2357-2368